# EUROPEAN PATENT APPLICATION

(11) **EP 3 818 925 A1**
(43) Date of publication of application: **12.05.2021**
(21) Application number: 20203990.5
(22) Date of filing: 27.10.2020
(51) Int. Cl.: A61B 5/00

(54) **FOOTWEAR AND SYSTEM FOR SENSORY TESTING OF FEET**

(30) Priority: 06.11.2019 TR 201917223
(71) Applicant: Arçelik Anonim Sirketi, 34445 Istanbul (TR)
(72) Inventor: CAKIN, Ilgaz, 34445 Istanbul (TR); HALATOGLU, Ugur, 34950 ISTANBUL (TR); TEKIN, Mehmet Ercan, 39450 ISTANBUL (TR)

(57) **Abstract**

It is described a footwear (1) for sensory testing of feet, the footwear comprising a flexible casing (2) for being worn on foot, a plurality of stimulators (3) for stimulating the skin of foot, a processing unit (4) for controlling the stimulators (3), and a connectivity module configured to establish data connection between the footwear (1) and a computing device. It is further described a system for and a method of sensory testing of feet.

## Description

The present invention relates to sensory testing of feet. The invention relates particularly to a footwear and a system for, and a method of sensory testing of feet.

The human nervous system can be adversely affected by diseases like cancer, rheumatism, diabetes or due to use of certain medicines. Degradation in nerves usually starts at points remotest from the brain, especially in feet. A very common symptom of nerve injuries or damages is numbness or loss of sensitivity in the part of the body concerned. Tests on functioning of nerves in the feet are therefore significant for diagnosis and monitoring of such diseases, for example, diabetes. One of these quantitative sensory tests is the Semmes-Weinstein monofilament (SWM) testing that measures response of a subject or patient to touching sensation of the monofilaments using a specific numerical quantity. The monofilament pressure of a predetermined force is applied to points on plantar and dorsal parts of the foot. The patient is asked whether he/she feels the touch and a sensory assessment is made upon recoding the responses, namely, to map out sensory loss.

In known techniques, the Semmes-Weinstein monofilament test is usually carried out by using a handheld probe device including a monofilament and an actuator. The probe device is controlled by a health specialist to apply the pressure to target points, and after each application recording patient's response to map out sensory condition of the tested foot. These techniques can only be exercised under supervision and by direct involvement of a health specialist. Besides, because of special medical equipment required, the test and assessment must take place at a clinic or hospital.

In the state of the art, United States patent application US2006178596 discloses a sensory testing system to determine sensory pressure thresholds. The system comprises a handheld probe including a test filament. Force is applied by the device to various test locations on a foot and a map of subject's extremity is generated using measured data.

United States patent application US2008193905 discloses a system for an automated palpation testing of patient's foot. For the purposes of automated testing, the patient puts his/her foot on a robotic device comprising an actuator controlled to apply specific forces to plantar.

The aim of the present invention is the realization of a footwear for sensory testing, wherein an accurate neuropathy test can conveniently be made at home, without presence of a health specialist. Another purpose of the present invention is provision of a footwear for sensory testing which can be conveniently used by a patient in sitting or lying position, as well as in standing position.

The aim of the present invention is achieved by the footwear explicated in claim 1, the system explicated in claim 10, and the method for sensory testing of foot explicated in claim 15. Further achievements have been attained by the subject-matters respectively defined in the dependent claims.

The footwear for sensory testing comprises a flexible casing for being worn on foot, a plurality of stimulators for stimulating the skin of foot, a processing unit for controlling the stimulators, and a connectivity module configured to establish data connection between the footwear and a computing device.

In a preferred embodiment, the casing comprises a sole region for at least partially covering the sole of the foot and an instep region for at least partially covering the instep of the foot. The stimulators are disposed on the sole region and on the instep region. In preferred embodiments, the casing further comprises lateral regions for at least partially covering lateral parts of the foot, and the footwear further comprises stimulators disposed on the lateral regions.

The flexible casing is preferably arranged to, when the footwear is worn on a foot, take the shape of the foot. The footwear may be in the form of a shoe or a sock. The casing may be made of, at least partially, textile or fabric. The material thereof is preferably synthetic and/or antibacterial. The textile may be, at least partially, electronic textile. Thus, the casing may include embedded electronics; such as stimulators, one or more processing unit, the connectivity module and electronic connections. The footwear may further comprise a power source for powering the processing unit and the stimulators.

In some embodiments, the stimulator comprises a pressure module for applying pressure to foot. The pressure module may comprise a monofilament for contacting the foot and a motor for moving the monofilament.

In some embodiments, the stimulator additionally or alternatively comprises a vibration module for applying vibration to foot. The vibration module (6) may also comprise a motor.

According to another aspect of the invention, there is provided a system for sensory testing. The system comprises a footwear as in any one of the embodiments described in the foregoing description, and a computing device which is configured to be connected to the footwear. The computing device is preferably arranged to receive feedback from a user (e.g. patient) in response to each stimulation applied by the stimulator.

The computing device may further be arranged to generate a sensory map of the foot subject to sensory test based on feedbacks. The sensory map may comprise information pertaining to sensitivity of the sole, at least one lateral region, and/or the instep of the foot. The sensory map may be a three-dimensional map.

According to another aspect of the inventions, there is provided a method of sensory testing of foot using the system as in any one of the embodiments described in the foregoing description. The method comprises wearing the footwear on a user's foot, starting the test, applying pressure on foot by means of the pressure module, receiving feedback from the user by means of the computing device, applying vibration on foot by means of the vibration module, receiving feedback from the user by means of the computing device, and generating a sensory map of the foot based on received feedbacks. The method may further comprise displaying the map and/or sending the map (and/or data thereof) to another computing device.

The footwear realized in order to attain the aim of the present invention is illustrated in the attached figures, where:
Figure 1 is a schematic illustration of the footwear according to an embodiment;
Figure 2 is a schematic cross-section side view of the footwear according to an embodiment, when worn on a foot;
Figure 3a is a schematic top view of the footwear according to an embodiment;
Figure 3b is a schematic bottom view of the footwear according to an embodiment;
Figure 4a is a schematic bottom view of a stimulator according to an embodiment;
Figure 4b is a schematic front view of a stimulator according to an embodiment.

The elements illustrated in the figures are numbered as follows:
1. Footwear
2. Casing
3. Stimulator
4. Processing unit
5. Power source
6. Pressure module
7. Vibration module

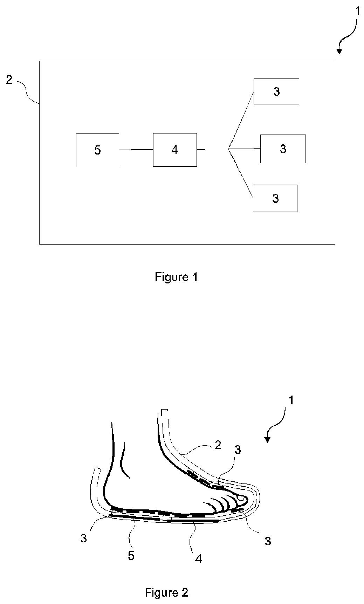

The footwear (1) for sensory testing of foot comprises a flexible casing (2) for being worn on foot, a plurality of stimulators (3) for stimulating the skin of foot, a processing unit (4) for controlling the stimulators (3), and a connectivity module configured to establish data connection between the footwear (1) and a computing device (Figure 1).

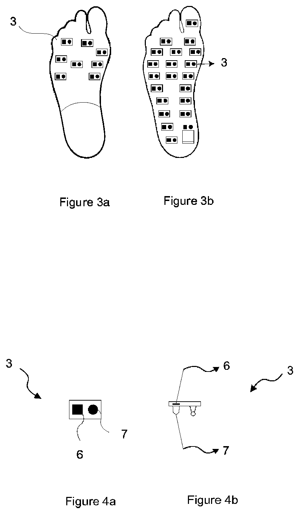

In a preferred embodiment, the casing (2) comprises a sole region for at least partially covering the sole of the foot and an instep region for at least partially covering the instep of the foot, and the footwear (1) comprises stimulators (3) disposed on the sole region and stimulators (3) on the instep region (Figure 2). The flexible casing (2) preferably takes the shape of the foot when worn. The sole region and stimulators (3) disposed thereon, when the footwear (1) is worn on a foot, at least partially cover the plantar of the foot (Figure 3b). Similarly, the instep region and stimulators (3) disposed thereon at least partially cover the dorsal of the foot (Figure 3a). In at some embodiments, the casing (2) further comprises lateral regions for at least partially covering lateral parts of the foot and the footwear (1) comprises stimulators (3) disposed on the lateral regions. Accordingly, the footwear (1) can perform sensory testing from various innervation territories of a foot, including medial plantar nerve (MPN), lateral plantar nerve (LPN), medial calcaneal nerve (MCN), saphenous nerve (SA), sural nerve (SU), superficial peroneal nerve (SPN) and deep peroneal nerve (DPN).

The stimulators (3) are preferably integrated to the casing (2). In embodiments, an array of stimulators (3) are arranged on the casing (2) in a grid structure, for example in an equally spaced arrangement. The stimulators (3) may be grouped into regions, for instance based on innervation territories of the foot. As a result, sensory measurement from a foot can be made for one or more specific nerve territory and/or in a specific order by employing respective group of stimulators (3) as appropriate.

The flexible casing (2) may be made of, at least partially, a synthetic and preferably antibacterial flexible material. The material can be any suitable textile, fabric, leather, etc. The footwear (1) may be in the shape of, for example, a shoe, a boot, or a sock.

In operation, the footwear (1) is electrically powered, for example, by means of a power chord from a power outlet or by a battery. The footwear (1) may thus further comprise a power source (5), such as a battery, for powering the processing unit (4) and the stimulators (3).

According to some embodiments, the footwear (1) is a 'smart garment'. The casing is made of or comprises electronic textile, wherein at least some of the electronic components are embedded. Embedded electronic components may include stimulators (3), the processing unit (4), the power source (5) and the connectivity module.

At least some of the plurality of stimulators (3) may comprise a pressure module (6) for applying pressure to foot. The pressure module (6) preferably comprises a monofilament or an equivalent needle like element for stimulating the foot by applying a contact force. In this embodiment, the pressure module (6) further comprises an actuator for moving the monofilament, such as a motor. During pressure stimulation, the filament can be moved up and down by the actuator. This movement is preferably controlled by the processing unit (4) according to requirements of the test, for example to perform a Semmes-Weinstein test.

At least some of the plurality of stimulators (3) may comprise a vibration module (7) for applying vibration to foot. The vibration module (7) may also comprise a motor to apply vibrations on the foot for testing purposes.

In embodiments, each stimulator (3) comprises a pressure module (6) and a vibration module (7) (Figure 4a, Figure 4b). However, at least some of the plurality stimulators (3) may comprise only a pressure module (6) or only a vibration module (7).

The present invention extends to a system for sensory testing of foot. The system comprises the footwear (1) according to any one of the embodiments described above, and a computing device configured to be connected to the footwear (1). During a sensory testing of foot, the computing device is in communication with the footwear (1) to send and receive signals and data as required. The footwear (1) and the computing device may be connected via wireless connection, for example by Bluetooth connection. The computing device may be a personal computer, a health kiosk, a handheld device, a tablet, a smart phone, etc. In an exemplary embodiment, the computing device is a mobile device comprising software, such as a dedicated mobile application for running a sensory test, in communication with the footwear (1).

In preferred embodiments of the invention, the computing device is arranged to receive feedback from a user in response to each stimulation applied by the stimulator (3). The user can be the patient on whom the sensory test is being made. The feedback provides an indication of whether the patient or subject sensed a respective stimulation, which can be pressure or vibration caused by a stimulator, applied on his/her foot or not. The feedback may be received via input means integrated or connected to the computing device.

The computing device may further be arranged to generate a sensory map of the foot subject to the test, based on received feedbacks. The sensory map is a visual representation of sense information regarding points and/or areas of the foot subject to testing. Thus, the map comprises information pertaining to sensitivity of the sole, at least one lateral region, and/or the instep of the foot. The sensory map may be a three-dimensional (3D) map, such as a 3D modelling of tested foot with sense information on respective points and/or regions thereof. The sense information, for example a colour-coded representation, may show whether and to what extent the subject or patient responds to, and hence feels, the stimulation applied at a certain location in his/her foot. The sensory map may be divided into sensory innervation territories of the foot, comprising regions corresponding to one or more of MPN, LPN, MCN, SPN, DPN, SU and SA. Accordingly, the sensory map may provide neuropathy information on sole, toes, instep and/or lateral parts of the tested foot.

The computing device may comprise a display means and may be arranged to display the sensory map. Additionally, or alternatively, the computing device may be configured to send the sensory map to a remote device, for example to a remote device associated with a health specialist for further examination.

The present invention further extends to a method of sensory testing of foot, using the system according to any one of the embodiments described above. The footwear (1) may be suitable for both right and left feet. In this case the test can be carried out separately for both feet, by using the same footwear (1). Otherwise, the footwear (1) can be manufactured in pairs, one for right and one for left foot. In this case, the test can be performed for both feet at one sitting. The method comprises wearing the footwear (1) on a foot to be tested and starting the test. The test may be started by a user command received via the computing device. The method further comprises applying a stimulation on foot. The stimulation may be pressure applied by means of the pressure module (6) or vibration applied by means of the vibration module (7).

Then, feedback regarding the subject's sense of the stimulation is received, preferably by means of the computing device. The feedback received is stored, preferably in a storing means comprised in the computing device. The sequence of stimulation, feedback and recording is repeated as many times and on desired parts of the foots as required, depending on specific purposes of the performed test. The sensory test may therefore comprise a pressure test only, a vibration test only, or both pressure and vibration tests. The method further comprises generating at least one sensory map of the foot based on received feedbacks. In at least some embodiments, the method also comprises displaying the sensory map on the computing device and/or sending the sensory map to another computing device.

Now an exemplary embodiment of the method will be described. A patient executes a sensory test on one of his foot, using a mobile phone as the computing device. The user wears the footwear (1), ensures that the mobile phone is connected to the footwear (1) and opens the mobile application for the sensory test on the mobile phone. The test will be performed for all parts of the foot and will comprise both a pressure and vibration testing. Via the application interface, user inputs a start command. A pressure module applies a predetermined pressure on the foot, the user selects on the mobile device whether he has sensed the pressure or not. The location of pressure module and the user feedback received therefore is stored. This is repeated for all pressure modules. Then, in a similar manner, vibration modules are activated one by one, receiving and storing user feedback for each respective vibration applied. Once all measurements are completed, a 3D sensory map of the foot is generated, showing the tested points and/or regions in sole, intake, toes and/or lateral parts of the foot together with indications of normal, no or weak sense associated therewith. The user may forward the sensory map to a clinic or directly to his doctor for further examination or an appointment.

In view of technical features and details explained above, the present invention proposes a footwear (1), a system and methods for sensory testing of foot, wherein the process of assessing neural functioning around the foot is significantly improved. More particularly, by employing the present invention a user can easily perform the sensory test unaccompanied by another person. The footwear (1) also allows the user to carry out the test at home, either in sitting, standing or lying position. Furthermore, by using a flexible casing (2) comprising stimulators (3) disposed on parts of the footwear (1) fitting onto upper and lateral parts of the foot, together with those on the sole portion, an extended area of the foot can be measured. As a result, the process of measuring and monitoring the functioning of nerves around the foot is not only facilitated but also improved.

## Claims

1. Footwear (1) for sensory testing, comprising
a flexible casing (2) for being worn on foot, the casing (2) comprising a sole region for at least partially covering the sole of the foot and an instep region for at least partially covering the instep of the foot,
a plurality of stimulators (3) for stimulating the skin of foot, wherein said stimulators (3) are disposed on the sole region and on the instep region,
a processing unit (4) for controlling the stimulators (3), and
a connectivity module configured to establish data connection between the footwear (1) and a computing device.

2. Footwear (1) according to claim 1, wherein the casing (2) further comprises lateral regions for at least partially covering lateral parts of the foot, and the footwear (1) comprises stimulators (3) disposed on the lateral regions.

3. Footwear (1) according to claim 1 or 2, wherein the casing (2) is made of textile.

4. Footwear (1) according to claim 3, wherein the textile is electronic textile.

5. Footwear (1) according to claim 4, wherein the textile comprises the stimulators (3) and/or processing unit (4) embedded therein.

6. Footwear (1) according to any one of the preceding claims, wherein the footwear (1) further comprises a power source (5) for powering the processing unit (4) and the stimulators (3).

7. Footwear (1) according to any one of the preceding claims, wherein the stimulator (3) comprises a pressure module (6) for applying pressure to foot.

8. Footwear (1) according to claim 7, wherein the pressure module (6) comprises a monofilament and a motor for moving the monofilament.

9. Footwear (1) according to any one of the preceding claims, wherein the stimulator (3) comprises a vibration module (7) for applying vibration to foot.

10. A system for sensory testing, comprising the Footwear (1) according to any one of the preceding claims and the computing device configured to be connected to the footwear (1)

11. The system according to claim 10, wherein the computing device is arranged to receive feedback from a user in response to each stimulation applied by the stimulator (3).

12. The system according to claim 11, wherein the computing device is further arranged to generate a sensory map of the foot based on feedbacks.

13. The system according to claim 12, wherein the sensory map comprises information pertaining to sensitivity of the sole, at least one lateral region, and/or the instep of the foot.

14. The system according to claim 13, wherein the sensory map is a three-dimensional map.

15. A method of sensory testing of foot using the system of any one of claims 10 to 14, wherein the method comprises:
wearing the footwear (1) on a user's foot,
starting the test,
applying pressure on foot by means of the pressure module (6),
receiving feedback from the user by means of the computing device,
applying vibration on foot by means of the vibration module (7),
receiving feedback from the user by means of the computing device, and
generating a sensory map of the foot based on received feedbacks.
